# EUROPEAN PATENT APPLICATION

(11) **EP 4 548 886 A1**
(43) Date of publication of application: **07.05.2025**
(21) Application number: 23845761.8
(22) Date of filing: 27.07.2023
(51) Int. Cl.: A61F 5/56, A61C 19/045

(54) **MANDIBULAR ADVANCEMENT SELECTOR**

(30) Priority: 28.07.2022 ES 202231264 U
(71) Applicant: Patricia Fernandez Sanjuan S.L.P, 28015 Madrid (ES)
(72) Inventor: FERNÁNDEZ SANJUÁN, Patricia, 28015 Madrid (ES); RÍOS LAGO, Marcos, 28015 Madrid (ES); CARBÓ BECH, Albert, 28015 Madrid (ES); ALONSO MERINO, Angel, 28015 Madrid (ES)
(74) Representative: Carlos Hernando, Borja
(86) International application number: PCT/ES2023/070488
(87) International publication number: WO 2024/023388

(57) **Abstract**

The present invention (mandibular advancement selector) relates to a device for measuring mandibular advancement and opening and to a simulator of the effect of a mandibular advancement device (MAD), assisting in the selection of patients who may be candidates to be treated for snoring, sleep apnoea syndrome or obstructive sleep apnoea (OSA) using a MAD. The selector of the invention allows the controlled opening and closing of a patient's jaws, following the natural rotation and opening movement of the mouth, thus preventing unnecessary stresses on the selector and the transfer of same to the jaws.

## Description

### Object of the invention

The present invention (mandibular advancement selector) relates to a device for measuring mandibular advancement and opening and to a simulator of the effect of a mandibular advancement device (MAD), assisting in the selection of patients who may be candidates to be treated for snoring, sleep apnoea syndrome or obstructive sleep apnoea (OSA) using a MAD. The device will preferably be used in combination with the DISE (*Drug Induced Sleep Endoscopy)* technique, in which patient's sleep is induced and the upper airway (UA) is observed with a nasopharyngoscope while the patient is sedated. Thus, it will be possible to visualise at what level the collapse occurs, what the pattern and degree of collapse is, as well as whether the UA is stabilised at one or more levels and to assess whether we are dealing with a potential patient who responds to treatment with mandibular advancement devices. This mandibular advancement selector can also be used in the dental clinic to record the intermaxillary relationship to be sent to the laboratory for fabrication of the final MAD for treatment.

The selector of the invention will simulate the effect of the MAD as it advances the jaw in small increments, in the range of millimetres, starting from a position of no advancement until it achieves, within the tolerable range of mandibular advancement, the elimination of UA collapses in the responding patient. It will also provide guidance on the amount of advancement required by each patient, minimising the number of unnecessary advancements. Likewise, the selector of the invention allows the controlled opening and closing of a patient's jaws, following the natural rotation and opening movement of the mouth, thus preventing unnecessary stresses on the selector and the transfer of same to the jaws.

### Description of the state of the art

There are different devices in the state of the art for controlling the mandibular advancement of a patient in the treatment of snoring and sleep apnoea syndrome or obstructive sleep apnoea (OSA), such as, for example, those which are disclosed in Spanish utility models with numbers ES1213585U, ES1220435U and ES1221934, as well as in the international application WO2019219998A1. The first three documents, in addition to detailing the problems associated with snoring and OSA, disclose mandibular advancement selectors that allow the controlled and regulated displacement of one part of the selector relative to the other due to the rack and pinion/sprocket system that guarantees said gradual displacement. There are also other devices that achieve sliding of one part relative to the other by manual displacement, overcoming the frictional forces between the two parts by pushing or pulling one of said parts. In these devices, sliding depends on the user's skill to overcome the friction between the two parts and achieve a controlled sliding. It should also be borne in mind that the patient often squeezes the jaws during sedation, which hinders the movement thereof, and mechanical aids may be necessary, such as the rack and pinion/sprocket system, which allows the transmission of forces to achieve precise forward and backward mandibular movements, overcoming the force exerted by the patient on the jaws.

It is also important to be able to lock the relative displacement or sliding between the two parts of the device by means of a braking/locking system that is also simple and convenient for the user of the device.

Moreover, devices are known in the state of the art that allow the separation of both jaws for opening the patient's mouth, but they do not simulate the natural opening of the mouth as they move one or both parts of the device in a strictly perpendicular manner with respect to the other to force the jaws apart, which, as is known, do not separate in a strictly perpendicular manner, but rotate one jaw with respect to the other about the temporomandibular joint.

The selector of the present invention achieves mandibular separation by helping this rotation from outside the mouth, thus preventing unnecessary stresses in the separation mechanism of the selector, as well as preventing the transmission of these unnatural stresses to the jaws. Opening and closing is achieved by gentle digital traction movements, exerted by the fingers of a single user's hand, accompanying a natural rotation and mandibular displacement movement while maintaining a tactile sensation of the pressure exerted on the jaw at all times.

Specifically, when the patient is sedated, the system that allows the mouth to be opened to see the effect of the change in the patient with vertical opening, it is desirable that this is done without forcing the temporomandibular joint. This opening can be done with tactile control of the force exerted by the user's hands so as not to strain said joint. The opening movement is usually performed while the jaw has been previously moved anteriorly, hence the importance of applying the opening stress directly with the tactile sensation of the user's hands. It is possible to detect whether a passive opening is being performed or whether resistance to movement is being put up and the joint position being explored may be forced. Whereas, if this stress is applied, for example, with a rack and pinion system, the detection of possible forces applied on the joint when it starts to be forced becomes less perceptible due to the arrangement of intermediate elements between the hands and the jaws. In short, it is a passive movement that should be performed when the mouth opening movement of the sedated patient is allowed, without forcing it.

Moreover, precision is required when measuring with the forks to record the intermaxillary relationship for manufacturing the MAD. The patient is awake and cooperative and controlled and regulated opening means can be used.

Other problems with state-of-the-art devices are the use of forks that do not adapt to the different anteroposterior dimensions of each patient's teeth, or of trays that adapt to the transverse size of each arch and maintain the chosen dimension stable.

The invention consists of a mandibular advancement measuring device, the embodiments of which solve the problems of the prior art.

### Description of the invention

The subject matter of the present invention is a mandibular advancement selector according to claim 1.

Specifically, the selector comprises:
- A first substantially straight segment, with a first end equipped with attachment means for attaching to a first mouth holder,
- A second segment able to be displaced in parallel with respect to the first segment and having a first end equipped with attachment means for attaching to a second mouth holder, and
- First displacement means for moving the second segment with respect to the first segment, achieving horizontal displacement of one jaw with respect to the other,
the second segment comprising:
- A substantially straight first base, with a first end and a second end, associated with the first displacement means,
- A curved guide, with an upper end and a lower end, emerging from the first end of the first base from the lower end of the curved guide, and
- A substantially straight second base, with a first end and a second end, joined at its second end to the curved guide by means of second displacement means, so as to be vertically distanced from the first segment, the curvature of the guide being such that, at the position of maximum distance between the second base and the first segment, the second end of the second base is further away from the first segment than the first end of said second base. The attachment means for attaching to the mouth holder, specifically to the second mouth holder, are arranged at the first end of the second base which is aligned with the first end of the second segment.

The first and second mouth holders comprise attachment means that are attached to the attachment means of the first and second segments. Said mouth holders are elements that are inserted into the patient's mouth and are displaced or actuated by the selector that is the subject of the invention when interacting with the patient's teeth. Preferably, said mouth holders are at least one of a tray to receive the jaws or a fork to be placed under the jaws or a support-guide for the incisors or a splint, attached to at least one of the two segments of the selector.

Typically, the two trays will be used together, one attached to the first segment and one attached to the second segment, so that one receives the upper arch, or upper jaw, and the other receives the lower arch, or lower jaw. Furthermore, the fork is used in conjunction with the support-guide for the incisors, so that the fork is attached to one of the segments, preferably the second segment, and the support-guide for the incisors is attached to the other segment, preferably the first segment. Sometimes, instead of a tray, fork or support-guide, a splint with the same attachment means for attaching to the selector could be used.

The selector achieves, by means of the curved guide, the mandibular separation helping the opening and rotation of one jaw with respect to the other from outside the mouth, i.e., accompanying the mandibular separation movement with the angulation in the opening of the device, avoiding a strictly vertical opening. This prevents unnecessary stresses on the selector separation mechanism and its components and prevents the transmission of these unnatural stresses to the jaws.

Preferably, the second displacement means between the second base and the curved guide, comprise a projection on the second base and consecutive holes in the curved guide to receive said projection, thus allowing gradual and controlled displacement of the second base with respect to the curved guide. Said displacement along the curved guide can be done with a single hand by placing one finger on a first digital support at the second end of the second base and placing another finger on a second digital support at the upper end of the curved guide. These second displacement means also allow, in addition to the gradual and controlled displacement of the second base with respect to the curved guide, a continuous displacement by applying greater force with the fingers. This continuous displacement is of particular interest for mandibular closing in situations where it is necessary to open or especially to close the mouth with a single gesture so that it can be easily and quickly removed from the mouth.

The curved guide preferably comprises means, more preferably a ruler, for determining the position of the second base with respect to said guide, and thus to know the degree of separation of the patient's jaws.

In order to achieve the displacement in both directions of the second segment with respect to the first segment, the first displacement means preferably comprise a sprocket that engages with a rack, the sprocket being associated with a manually operated wing nut that controls the rotation of said sprocket in a regulated manner in order to determine the exact displacement (in an anterior or posterior direction) while the displacement is carried out in a gradual and sustained manner to the jaws.

Likewise, in order to secure the position of the second segment with respect to the first segment, the first displacement means comprise a braking/locking mechanism which prevents the rotation of the sprocket and thus the displacement of the second segment with respect to the first segment. Preferably, the braking/locking mechanism comprises:
- the sprocket associated with the wing nut by means of a pivot of the wing nut inserted in a central housing of the sprocket, thus achieving a coaxial connection between the two,
- a pin, consisting of a first stem which passes through and is associated with the pivot of the wing nut, and which is attached to a second stem, being coaxial with each other and with the sprocket, said pin being movable between two positions on the inside of the wing nut, and
- a friction element, preferably an O-ring, but could also be an elastomeric ring, a silicone tube, a chicken-rubber or similar, located on the second stem.

By means of the above system, in one of the two positions of the pin, the friction element, preferably an O-ring, makes contact with an element of the first displacement mechanism, preventing the rotation of the wing nut and therefore the displacement of the second segment with respect to the first segment. In the second position of the pin, the friction element, or O-ring, does not make contact with or rub against any element, and relative displacement between the two segments is possible.

Alternatively, the braking/locking mechanism comprises:
- the sprocket associated with the wing nut by means of a pivot of the wing nut inserted in a central housing of the sprocket, thus achieving a coaxial connection between the two,
- a pin, consisting of a first stem which passes through and is associated with the pivot of the wing nut, and which is attached to a second stem, being coaxial with each other and with the sprocket, said pin being movable between two positions on the inside of the wing nut, and
- a locking assembly, consisting of a ring with teeth therein and a sprocket, the toothed ring being located on an element of the first displacement mechanism and the sprocket being arranged on the second stem.

By means of this mechanism, in one of the two pin positions, i.e. locking or braking position, the sprocket of the second stem is inserted in the toothed ring of the element of the first displacement mechanism, preventing the rotation of the wing nut and therefore the displacement of the second segment with respect to the first segment. In the second position of the pin, the sprocket of the second stem is outside the toothed ring, and relative displacement between the two segments is possible.

Preferably, the rack of the first displacement mechanism is located on the second base of the second segment, while the sprocket is arranged below the first segment and fixed thereto, so that when the wing nut is rotated, it drags the sprocket, which in turn causes the rack, and therefore the second base, to move relative to the first segment.

The attachment means for attaching each mouth holder allow the attachment and separation of said mouth holder (tray, fork or support-guide) from the corresponding segment with a single hand, and preferably, allows both the attachment and detachment of each mouth holder on its respective segment of the selector at the same time. Thus, the user can attach and detach both mouth holders (trays, forks, or support-guides) at the same time. The attachment means preferably comprise an external pushbutton, which actuates a flange, arranged at one end of the mouth holder (tray, fork or support-guide), which is inserted into, or exits from, a housing arranged at the end of the segment. In this way, when the pushbutton is pressed, the flange detaches from the housing arranged at the end of the segment, thus enabling the mouth holder (tray, fork or support-guide) to separate. Alternatively, the attachment means could be arranged in the opposite manner, i.e. the pushbutton and flange on the selector segment and the housing on the mouth holder (tray, fork or support-guide).

The invention also relates to a mouth holder (tray, fork or support-guide) with other particular characteristics in addition to those related to the attachment and detachment thereof from the selector of the invention.

Specifically, both the fork and the support-guide may comprise adaptation means for adapting to the anteroposterior dimensions of the patient's teeth. Said adaptation means comprise at least two parallel flanges arranged perpendicularly to a horizontal surface of the fork or the support-guide from which the flanges can be separated. Thus, if these anteroposterior dimensions of the teeth are larger, more flanges must be removed to ensure that the teeth reach the horizontal surface of the fork or the support-guide.

Preferably, the tray may also comprise adaptation means for adapting to the transverse size of the patient's arch. Said adaptation means comprise a fastening in the central area of the tray, comprising a central groove which divides the same into two symmetrical portions or sides and which allows the first and second sides to be moved closer or further apart, widening or reducing the angle of separation between the same to thus adapt to the dimensions of the arch. Preferably, said fastening is a tongue with a lug perpendicular thereto located on one side of said tray which is inserted into at least one of at least two holes arranged on the other side of said tray. The side with the holes may also include a hollow or recess to house the flange on the other side of the tray.

Preferably, the first segment of the selector comprises a ruler to verify the offset distance of the second segment with respect to said first segment. Said ruler is arranged at the rear end of said first segment, opposite to the end where the mouth holder (tray, fork or support-guide) is or can be attached. Preferably, a fixed transparent ruler is arranged at the rear end of said first segment. Below said transparent ruler, an unrelieved marking slides which determines the end of each tolerable limit movement of the protrusive and retrusive mandibular position. Preferably, to determine the mandibular movement, the selector in turn comprises two sliding parts on the first segment, which the user will position to coincide with each limit movement of the jaws in order to delimit the tolerable anteroposterior movement in each patient. These parts will remain stable during scanning, regardless of the movements made, and only with a new intentional manipulation will they move to another position for a new recording. In an alternative embodiment, in order to determine the tolerable limit movements of protrusive and retrusive mandibular position, the selector comprises two annular parts inside of which the second end of the first segment is inserted. Said annular parts are friction-fitted onto the first segment so that a first annular part determines the limit of the protrusive position and a second annular part determines the limit of the retrusive mandibular position. To ensure fit and friction between the first segment and the annular parts, the annular parts preferably have inner projections which may be made of a different material than the first segment so that friction is increased. By increasing the friction between the two components, it is possible to prevent accidental displacement of said annular parts, which could lead to the modification of the limit positions and thus pose a risk to the patient.

Alternatively, said ruler is removable and interchangeable with another ruler. In this way, in addition to being able to use different measuring scales or units, it is also possible to write down a patient's measurements directly on the ruler and subsequently insert a clean ruler and use the mandibular advancement selector with a new patient, saving the measurements taken for each patient, as they have been written down on each ruler.

The ruler is preferably 4 cm (from -20 mm to +20 mm) in total, which allows the maximum protrusive to be measured without limitations (within the range of anteroposterior movement for humans).

The preferred materials for the selector of the present invention are aluminium and/or machined steel. Moreover, injection moulded plastic can also be used for the wing nut. Also, the forks and trays are made of injected plastic.

### Brief description of the figures

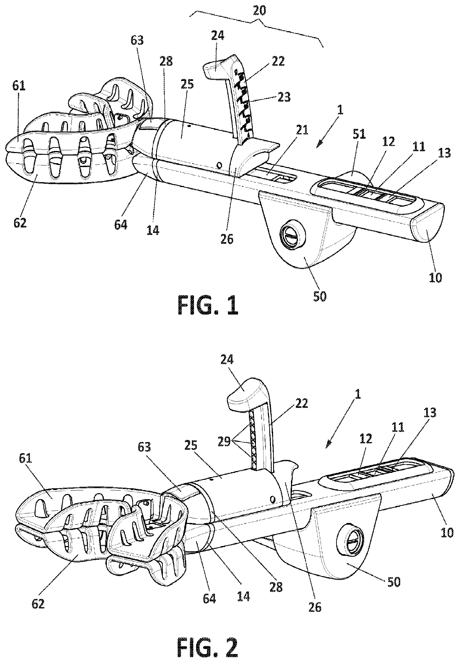

To facilitate the understanding of the present invention, the following figures are attached: Figure 1 shows a rear perspective view of the mandibular advancement selector of the invention with two trays.
Figure 2 shows a rear perspective view of the selector of figure 1.
Figure 3 shows an upper view of the selector of figure 1.
Figure 4 shows a rear view of the selector of figure 1.
Figure 5 shows an exploded view of the selector of figure 1.
Figure 6 shows an exploded view of the selector of figure 1 without the first segment.
Figure 7 shows a longitudinal cross-sectional view of the selector of figure 1 according to the B-B cut lines in figure 8.
Figure 8 shows a cross-sectional view of the selector of figure 1 according to the A-A cut lines in figure 7.
Figure 9 shows a perspective view of the mandibular advancement selector with a splint at the end of the segments.
Figure 10 shows a lower perspective view of the selector in figure 9.
Figure 11 shows an upper perspective view of the selector in figure 9.
Figure 12 shows the selector of figure 9 with the detached splints of the first and second segment from an upper perspective.
Figure 13 shows the selector of figure 9 with the detached first and second segment splints from a lower perspective.
Figure 14 shows a longitudinal cross-sectional view of the selector in figure 9.
Figure 15 shows a view of the selector of figure 1 in use, with one user's hand rotating the wing nut to move the second segment relative to the first, since, in use, the selector is inserted into the patient's mouth and held by the same.
Figure 16 shows the selector of figure 1 in use, with the second segment offset to the rear of the first segment.
Figure 17 shows the selector of figure 1 in use, with the hand's thumb placed below the first digital support at the second end of the second base of the second segment and the index finger, slightly flexed and arranged on the second digital support at the upper end of the curved guide, to start lifting the second base, and the tray associated with said second base, along the curved guide, the second base of the second segment being still next to the first segment.
Figure 18 shows the selector at a subsequent step to that in figure 17, with the second base halfway along the length of the curved guide and therefore partially separated from the first segment and determining a mandibular separation.
Figure 19 shows the selector at an even later step than that in figure 18, with the second base at the upper end of the curved guide and therefore with the maximum possible mandibular separation that the selector can cause.
Figure 20 shows the next step after that in figure 19, prior to repositioning the second base of the second segment next to the first segment by lowering the second segment along the curved guide, for which purpose the hand's thumb rests above the first digital support at the second end of the second base of the second segment and the index finger is slightly flexed and arranged below the first segment.
Figure 21 shows the next step after that in figure 20, when the second segment has descended all at once until meeting the first segment driven by the hand's thumb.
Figure 22 shows figure 17 from another point of view, where the wing nut can be seen being actuated.
Figure 23 shows the selector inserted in a patient's mouth and one hand handling the vertical displacement of the second base.
Figure 24 shows two trays in perspective, upper and lower, showing, in the centre of the upper arch, the adaptation means for adapting to the transverse size of the patient's arch.
Figure 25 shows an upper view of the upper tray with the adaptation means for adapting to the transverse size of the patient's arch.
Figure 26 shows an exploded view of a second embodiment of a selector with an alternative braking/locking mechanism.
Figure 27 shows a partial cross-sectional perspective view of the second embodiment of the selector, with the braking/locking mechanism deactivated.
Figure 28 shows a partial cross-sectional perspective view of the second embodiment of the selector, with the braking/locking mechanism activated.
Figure 29 shows a transversal view of the braking/locking mechanism.

### Description of preferred embodiments

A detailed description of the preferred embodiments of the present invention, a mandibular advancement selector, is given below, in accordance with the figures cited.

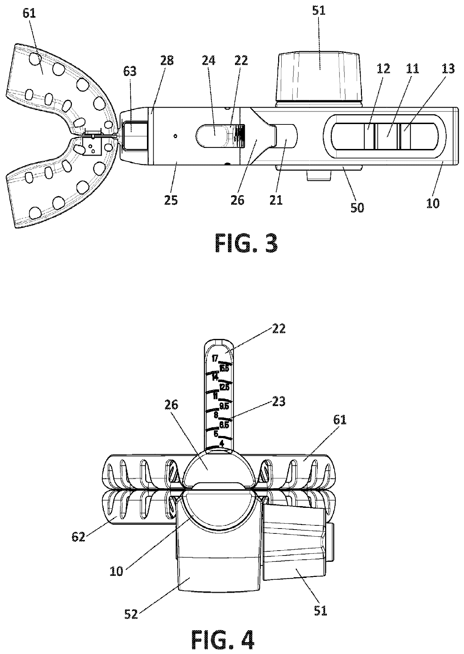

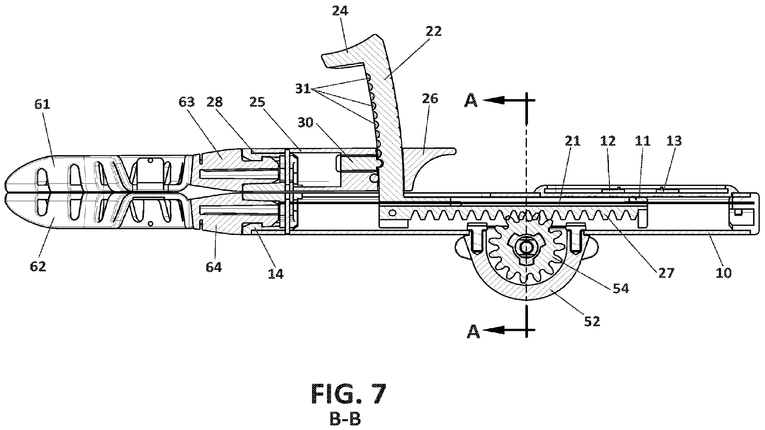

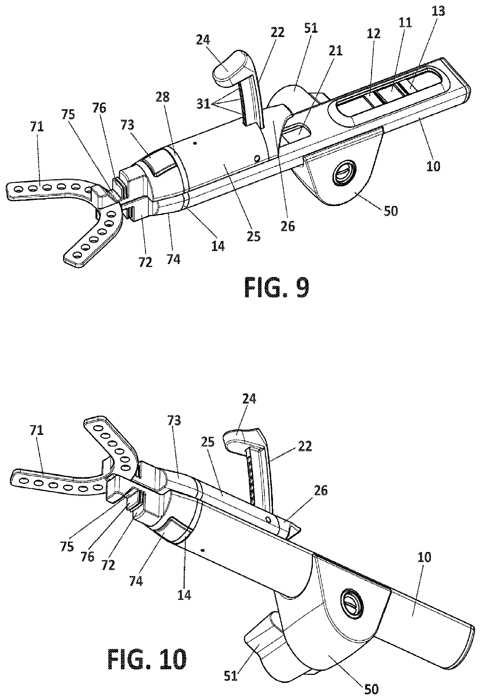

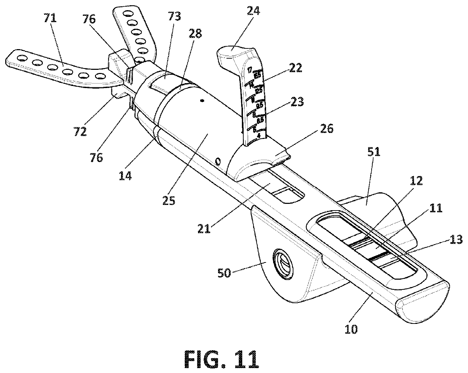

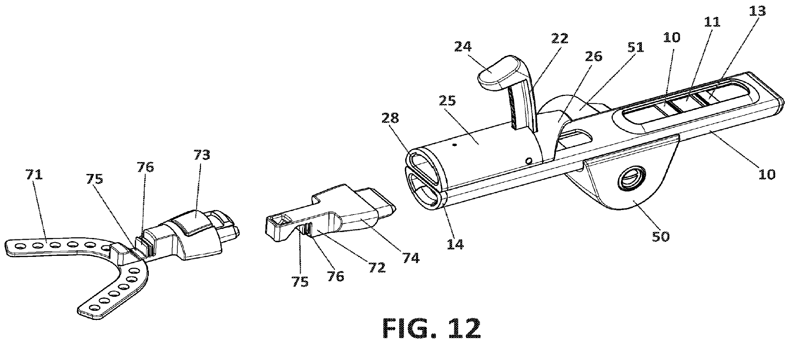

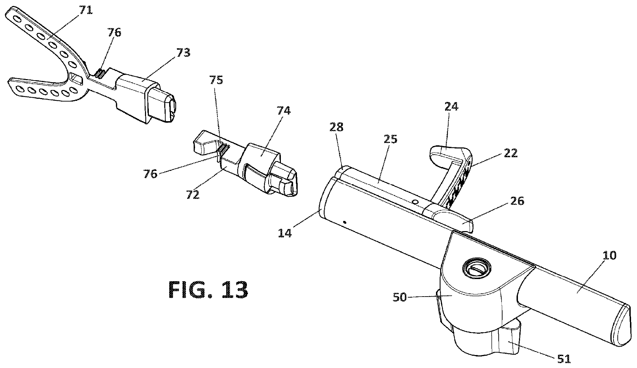

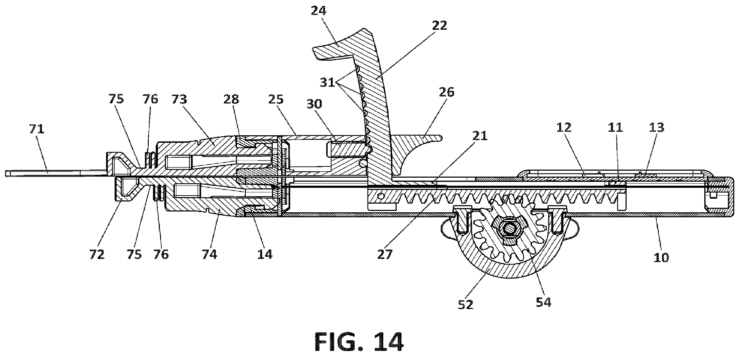

Figures 1 to 8 show a mandibular advancement selector (1) with two trays (61, 62) attached, as mouth holders, and in a removable manner from the selector, to one end of said selector (1). The selector (1) comprises a first substantially straight segment (10) with two ends, a first end equipped with first attachment means for attaching (14) to a first mouth holder, specifically a first tray (62), or lower tray, and a second free end opposite to the first end. The first attachment means (14) also serve for attaching a fork (see figures 9 to 14) as a mouth holder. The first tray (62) has first attachment means (64) complementary to the first attachment means (14) of the free end of the first segment (10).

The selector (1) comprises a second segment (20) that can be displaced with respect to the first segment (10), and as well as the first segment (10), comprises a first end equipped with attachment means for attaching (28) to a second mouth holder, specifically a second tray (61), or upper tray, with attachment means (63) complementary to the previous ones. The second attachment means (28) are also used for attaching a support-guide of the lower incisors (see figures 9 to 14). Both the first (10) and second (20) segment are associated with each other by means of first displacement means (50) to achieve the movement of the second segment (20) with respect to the first segment (10). Said displacement means are preferably a system consisting of a sprocket or pinion (54) and a rack (27), so that when the sprocket or pinion (54) rotates, the rack (27) moves. Alternatively, the first displacement means (50) can be elements protruding from the first (10) and second (20) segments to be manually pushed by the user, overcoming the frictional forces between the two segments. Likewise, said first displacement means (50) may comprise external elements that assist the relative displacement thereof, such as small electric motors. Said first displacement means (50) allow a regulated and controlled displacement of the second segment (20) with respect to the first segment (10).

The second segment (20) comprises a first substantially straight base (21), with a first and a second end opposite to each other and associated with the first displacement means (50) of the selector (1) and therefore associated with the first segment (1) of the selector (1). A curved guide (22) emerges from the first end of said first base (21). Said curved guide (22) has an upper end (24) and a lower end connected to the first base (21). A second substantially straight base (25) with a first end and a second end (26), through which it is associated or joined to the curved guide (22), is arranged on said curved guide (22). Second displacement means are provided between the curved guide (22) and the second base (25) in order to move said second base (25) along the curved guide (22) between the upper end (24) of said curved guide (22) and the lower end of said curved guide (22), which is equivalent to the contact of the second base (25) with the first segment (10). The second end (26) of the second base (25) is suitably shaped to be pushed by a user's finger.

Thus, due to the curvature of the curved guide (22), when in the position of maximum distance between the second base (25) and the first segment (10), that is, with the second end (26) of the second base (25) close to the second end (24) of the curved guide (22), the second end (26) of the second base (25) is further away from the first segment (10) than the first end where the second attachment means (28) of said second base (25) are located. This is due to the curvature of the guide (22) which is inclined towards the first end of the selector (1) where the mouth holders, i.e., trays (61, 62), fork (71) or support-guide (72), are attached. The curved guide (22) has adjustments that indicate the position of the second base (25) with respect to the first segment (10).

By means of this opening system, which is not strictly vertical, mandibular separation is achieved in a natural manner, reproducing a mouth's opening, and thus helping the opening and rotation of one jaw with respect to the other from outside the mouth by accompanying the movement of mandibular separation with the angulation in the opening of the device.

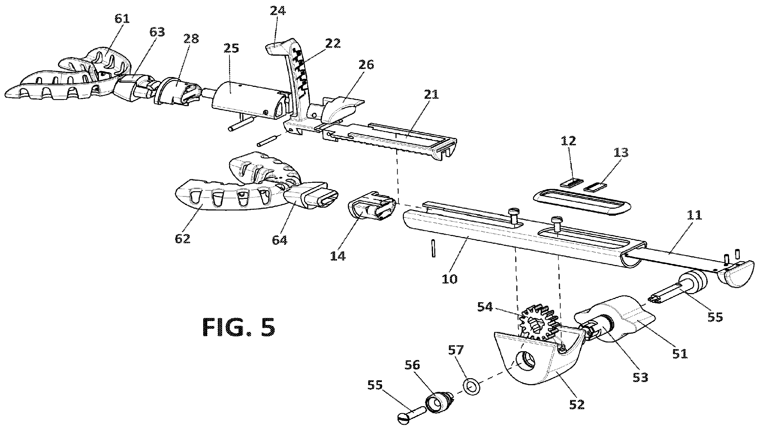

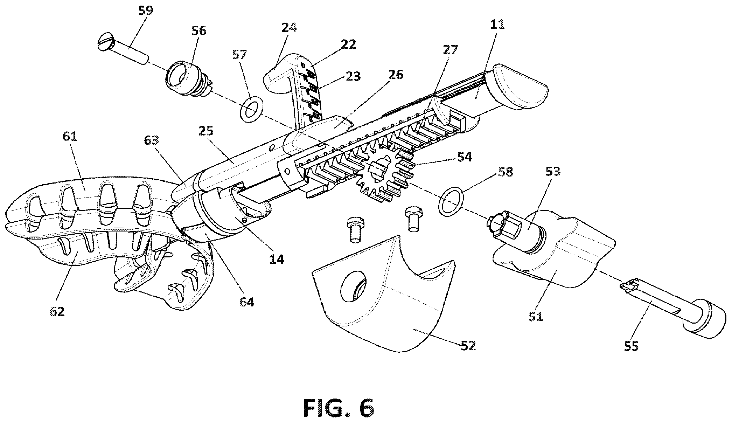

Figures 5 to 7 show a first displacement system of the second segment (20) with respect to the first segment (10), comprising a sprocket or pinion (54) and a rack (27). Figures 5 and 6 show an exploded view of the selector (1) showing the different components thereof. Specifically, it can be seen how the first displacement means (50) have the sprocket (54) which engages with the rack (27), located in the lower portion of the first base (21) of the second segment (20), and with the sprocket (54) associated with a wing nut (51) by means of a pivot (53) which is inserted into a hole at the centre of the sprocket (54) by means of appropriate and coaxial connection means, for example, complementary shapes between the inside of the central hole of the sprocket (54) and the outside of the pivot (53) of the wing nut (51). The sprocket (54) is concealed inside a housing (52). By rotating the wing nut (51), the rack (27) and therefore the second segment (20) are displaced with respect to the first segment (10) in a controlled and regulated manner. The wing nut (51) has a contact or friction element, preferably in the form of an O-ring (58), to regulate the rotation thereof, which is arranged between the pivot (53) and the housing (52).

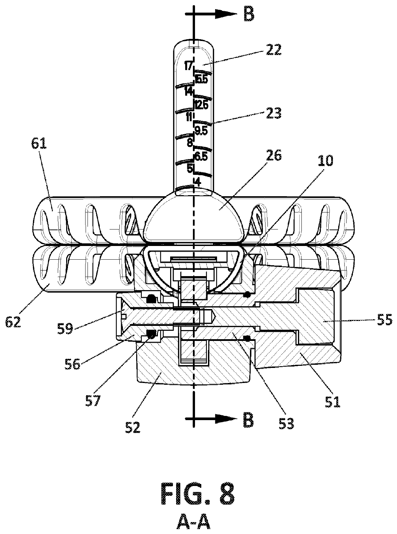

To fasten the position of the second segment (20) with respect to the first segment (10) it is necessary to have a braking/locking system (figure 8) to prevent the rotation of the sprocket (54) and therefore the displacement of the second segment (20) with respect to the first segment (10). The braking/locking mechanism preferably comprises a pin (59), consisting of a first stem (55) which passes through and is associated with the pivot (53) of the wing nut, and which is attached to a second stem (56), being coaxial with each other and with the sprocket (54). Said pin (59) is movable between two positions on the inside of the wing nut (51), one position in which the wing nut (51) can rotate and another in which rotation is not possible. Thus, when the pin (59) is located inside the second stem (56) and is viewed or protrudes from the side of the wing nut (51), a friction element, in the form of an O-ring (57) associated with the second stem (56) rubs against an element of the first displacement mechanism, for example with the housing (52), preventing the rotation of the wing nut (51) and therefore preventing the displacement of the second segment (20) with respect to the first segment (10). On the other hand, in a second position, the O-ring (57) does not rub against any element and therefore the sprocket (54) can rotate.

The second displacement means make it possible, as mentioned above, to move the second base (25) of the second segment (20) along the curved guide (22) and with respect to the first segment (10). Preferably said displacement means comprise a projection (30) on the second base (25) and consecutive holes (31) in the curved guide (22) to receive said projection (30). To achieve the displacement of the second base (25), the same is pulled or pushed by a user's finger placed on the second end of the second base (26) acting as a digital support (26) and another finger of the same hand rests on the upper end (24) of the curved guide (22) acting as a second digital support (24). Thus, when the first digital support (26) is pulled, the projection (30) comes out of one hole (31) and enters another hole (31) as it is slightly compressed to ensure its insertion into the holes (31). This system allows a controlled and regulated displacement of the second base (25) with respect to the curved guide (22), as well as a continuous displacement between both ends of the curved guide (22), depending on the use requirements of the selector (1). On the opposite side of the curved guide (22) to where the holes (31) are located, the ruler is provided to know the position of the second base (25) with respect to said guide (22).

Alternatively (not shown), projections could be provided on the curved guide (22) and a hole in the second base (25), so that when said second base (25) is moved, the various projections on the curved guide (22) are inserted into the hole in the second base (25).

Next to the second end of the first segment (10) a fixed transparent ruler (11) is provided to determine the patient's jaw movement. Below said transparent ruler (11), an unrelieved marking slides which determines the end of each tolerable limit movement of the protrusive and retrusive mandibular position. To determine the maxillary movement, the selector (1) comprises two sliding parts (12, 13) on the first segment (10), which the user will position to coincide with each limit movement of the jaws in order to delimit the tolerable anteroposterior movement in each patient. These parts (12, 13) will remain stable during scanning, regardless of the movements made, and only with a new intentional manipulation will they move to another position for a new recording.

Figures 9 to 14 show a selector (1) with a fork (71) as a mouth holder, attached to the first end of the second base (25) of the second segment (20). Said attachment is made by means of attachment means (28) located at said first end and attachment means (73) located on the fork (71). Said attachment means, as well as those used for the trays (61, 62) of figures 1 to 8, are complementary to the attachment means (14, 28) arranged at the first ends of the first segment (10) and second segment (20). When using a fork (71) attached to the second segment (20), a support-guide for lower incisors (72) is attached to the first segment (10) with the same attachment means, thus securing the position of the lower arch.

Preferably, said attachment means allow the removal with one single hand and at the same time of the dental holders, i.e., the fork (71) and the support-guide (72) or the two trays (61, 62). For this purpose, preferably, the attachment means (73, 74, 63, 64) of the dental holders, fork (71), support-guide (72) or trays (61, 62), comprise a projection solidly attached to a flange which is inserted in an internal housing in the attachment means (14, 28) of the first (10) and second segment (20) of the selector (1). Thus, when the projection is pressed, the flange lowers and comes out of the inner housing, enabling the removal of the dental holders, i.e., the fork (71), support-guide (72), trays (61, 62) or even splints, from the first (10) and second (20) segments.

Figures 9 to 14 also show a fork (71) and a support-guide (72) with adaptation means for adapting to the anteroposterior dimensions of the patient's teeth, in the case of the fork (71) for adaptation to the width of the upper incisors and in the case of the support-guide (72) for adaptation to the width of the lower incisors. Preferably, said adaptation means are at least two parallel flanges (76) arranged perpendicularly to a horizontal surface (75) of the fork (71) and of the support-guide (72) from which the flanges (76) can be separated. Thus, the fork (71) and the support-guide (72) adapt to the width of the patient's incisors, teeth that will be placed on said horizontal surface (75).

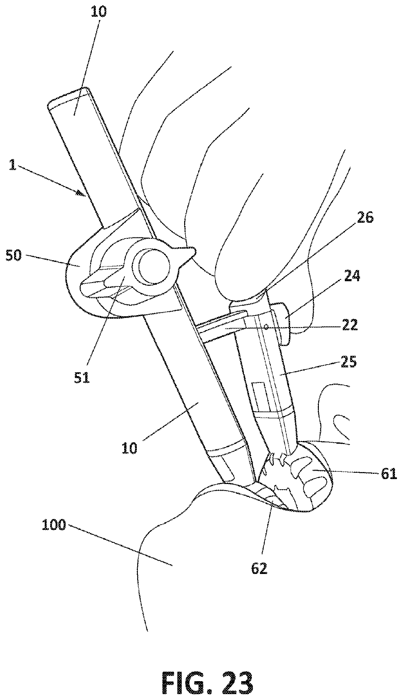

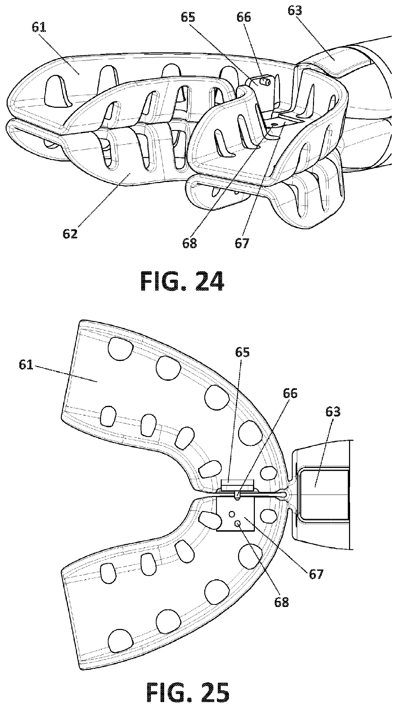

Figures 23 and 24 show a tray (61 , 62) having adaptation means to the transverse size of the patient's arch, wherein only one or both of the two trays (61, 62) may incorporate said adaptation means. The trays (61, 62) preferably have a U-shaped cross-section with two vertical walls and a substantially horizontal bottom, so that the dental arches are inserted therein. The tray (61, 62) has a central groove, joined by at least one of the vertical walls, which divides the tray into two symmetrical parts or sides and allows the sides to be separated by varying the angle of separation between the sides when pivoting against the joining wall, to adapt the same to the transverse size of the patient's arch. In order to fasten this position, in the vicinity of the groove and to block the possible opening of the groove, there is a fastening which allows the two sides of the tray to be moved closer together or further apart. The fastening comprises a tongue (65) on a first side of the tray, which pivots with respect thereto and which incorporates a lug (66), said lug being connected to one of at least two holes (68) arranged on the second side of the tray. This second side preferably has a recess or hollow (67) to receive the tongue of the first side. By these means, the tray can be adapted to the patient's jaw.

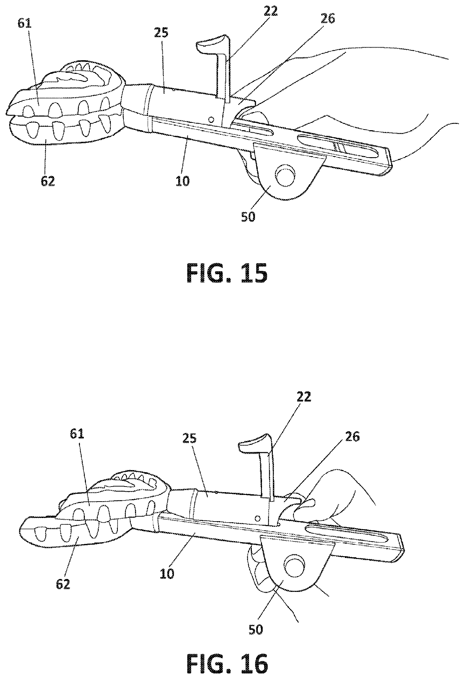

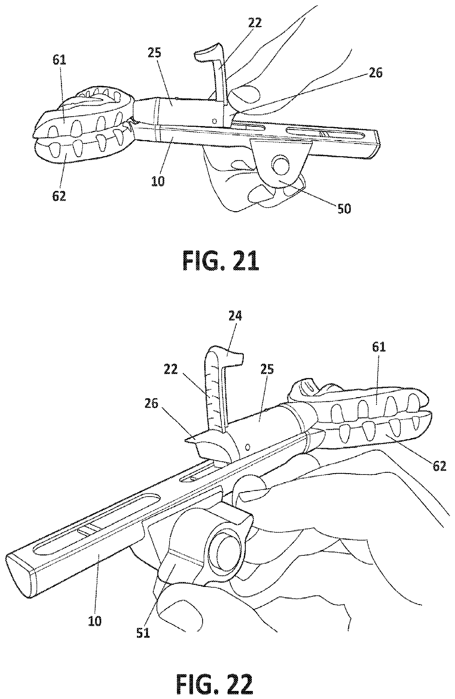

Figures 15 to 22 show how the selector (1) of the invention is used. In figures 15 to 22, it is understood that the trays (61, 62) are located inside the patient, although they are not shown for ease of representation, although figure 22 does show the selector (1) with the trays (61, 62) in a patient's mouth and each dental arch inserted in each of said trays (61, 62). When the dental holders, trays (61, 62), fork (71) or support-guide (72) are in the patient's mouth, the selector (1) can be operated with one single hand. Figure 15 shows a view of the selector (1) of Figures 1 to 8 in use, with one user's hand rotating the wing nut (51) to move the second segment (20) with respect to the first segment (10), moving one jaw with respect to the other when moving the second tray (61) with respect to the first tray (62). Figure 16 shows how the second segment (20) is offset towards the rear portion of the first segment (10) when the handle (51) is rotated by hand.

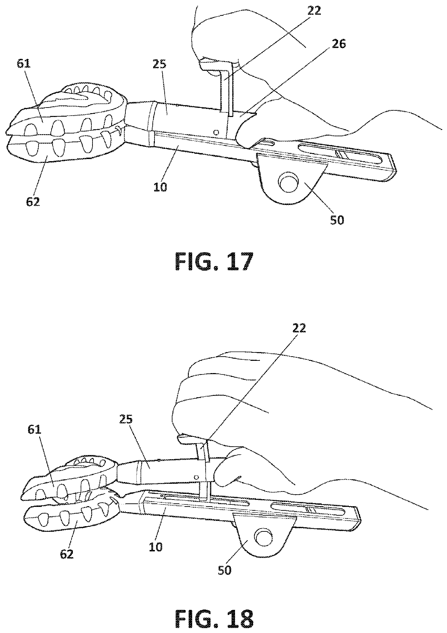

Figure 17 shows the selector (1) with the two trays (61, 62) aligned, i.e. without displacement of the second segment (20) with respect to the first segment (10) and prepared for the second base (25) to be raised and separated with respect to the first segment (10). For this purpose, the thumb of one user's hand is placed below the first digital support (26) of the second base (25) of the second segment (20) and the index finger of the same hand is slightly flexed and arranged on the second digital support (24) at the upper end of the curved guide (22), the second base (25) of the second segment (20) still being next to the first segment (10). From this position the second base (25), and the tray (61) associated with said second base (25), is raised along the curved guide (22), to, for example, an intermediate point of the curved guide (22) as shown in figure 18, with the second base (25) halfway along the length of the curved guide (22) and therefore partially separated from the first segment (10) and determining a separation between the upper and lower jaws. Said position could be maintained due to the second displacement means between the second base (25) and the curved guide (22).

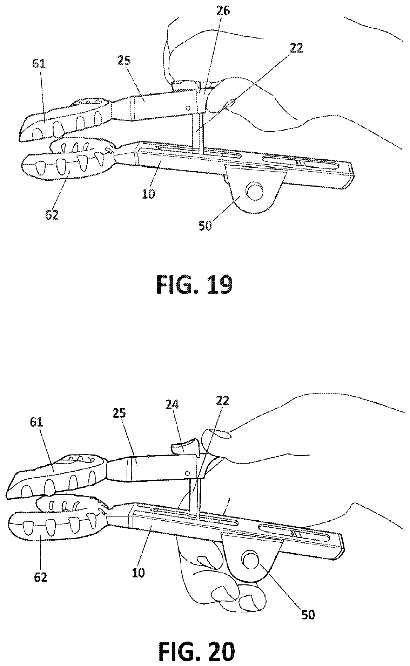

Figure 19 shows the selector (1) in an even later step than that in figure 18, with the second base (25) at the upper end of the curved guide (22), with the first digital support (26) and the second digital support (24) close to each other, determining the maximum possible jaw separation that the selector (1) can cause.

Figure 20 shows a subsequent step after that of figure 19, before starting to lower the second base (25) along the curved guide (22), and as a limit, repositioning the second base (25) of the second segment (20) next to the first segment (10). For this movement, the hand's thumb rests above the first digital support (26) on the second end of the second base (25) of the second segment (20) and the index finger of the same hand, slightly flexed and arranged below the first segment (10), so that one hand continues to hold the selector (1). From this position and with this hand grip, the user can either lower the second base (25) little by little, using the second displacement means, or lower the second base (25) directly and in a single movement until reaching the position shown in figure 21, when the second segment (25) has been lowered at once, driven by the thumb, until it meets the first segment (10).

Figure 22 shows the selector (1) inserted in the mouth of a patient (100) and a hand handling the vertical displacement of the second base (25) with respect to the curved guide (22) and thus controlling the separation between the upper and lower jaw of the patient (100).

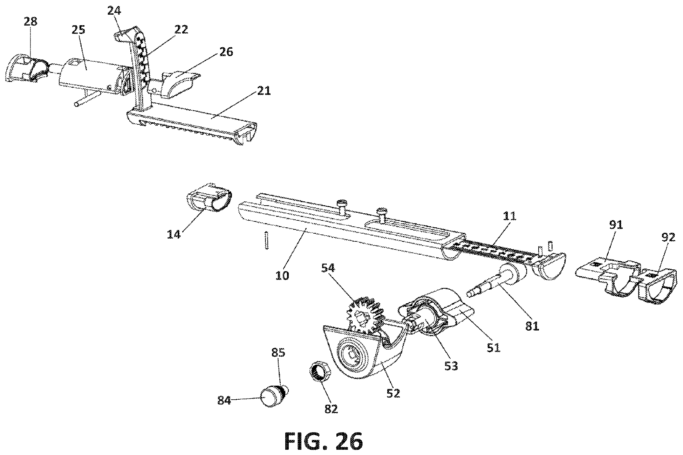

Figures 26 to 29 show a second embodiment of a mandibular selector of the invention. Specifically, this second embodiment incorporates an alternative braking/locking mechanism to the one previously described, as well as alternative parts that limit the limit positions of the patient's mandibular movement. A selector according to the present invention could have any of the two braking/locking mechanisms, as well as any other not described, as well as any of the mandibular movement limiting parts, as well as others not being described.

Figure 26 shows an exploded view showing the components of the alternative braking/locking mechanism for fastening the position of the second segment (20) with respect to the first segment (10). This alternative braking/locking mechanism (Figure 26, 27, 28 and 29) prevents, as well as the previously described braking/locking mechanism, the rotation of the sprocket (54) and therefore the displacement of the second segment (20) with respect to the first segment (10). This alternative braking/locking mechanism preferably comprises a pin consisting of a first stem (81) which passes through and is associated with the pivot (53) of the wing nut (51), and which is attached to a second stem (84), being coaxial with each other and with the sprocket (54). The first stem (81) and second stem (84) are preferably joined by a thread and an adhesive. Said pin is movable between two positions on the inside of the wing nut (51), one position in which the wing nut (51) can rotate and another in which rotation is not possible. To prevent the blade from turning, the braking/locking mechanism comprises a locking assembly consisting of a ring (82) with teeth therein and a sprocket (85), the toothed ring (82) being located on an element of the first displacement mechanism, e.g. on the inside of the housing (52), and the sprocket (85) being arranged on the second stem (84). Thus, by means of this mechanism, in one of the two positions of the pin, or locking or braking position, the sprocket (85) of the second stem (84) is inserted in the toothed ring (82) of the housing (52), preventing the rotation of the wing nut (51) and therefore the displacement of the second segment (20) with respect to the first segment (10) of the selector. In the second position of the pin, the sprocket (85) of the second stem (84) is outside the toothed ring (82), and relative displacement between the two segments (10, 20) is possible.

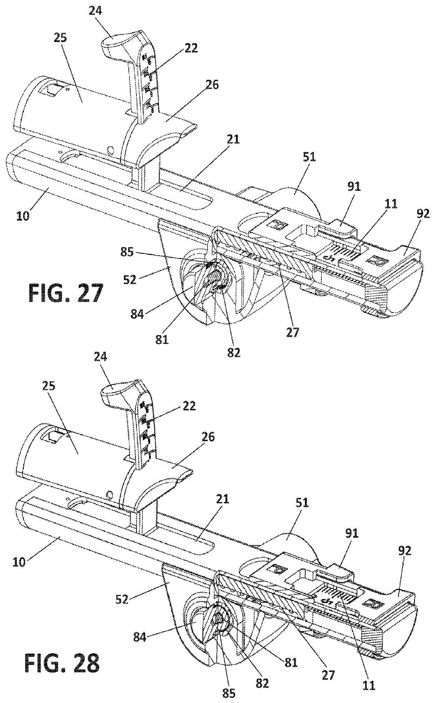

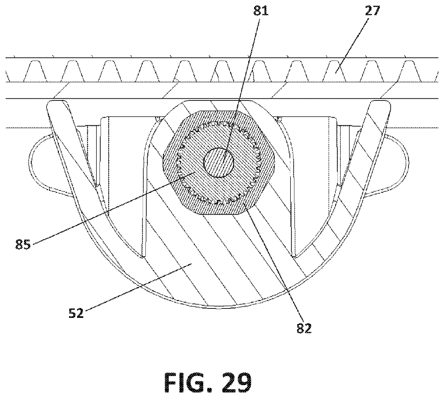

Figure 27 shows the selector with the previous braking/locking mechanism deactivated, i.e., enabling the rotation of the wing nut (51) and therefore the relative movement of both segments (10, 20), while figure 28 shows the selector with the braking/locking mechanism activated, i.e., preventing the rotation of the wing nut (51) and consequently the relative movement between both segments (10, 20). Figure 29 shows a cross section of the locking assembly showing in detail the configuration of the toothed ring (82) and the sprocket (85) of the second stem (84). Preferably, in the spaces between the teeth of the toothed ring (82), two teeth of the sprocket (85) are inserted, thus achieving a greater number of locking positions of the wing nut (51) and, by extension, a greater number of locking positions of the second segment (20) with respect to the first segment (10).

These figures (26 to 29) also show the parts (91, 92) that determine the tolerable limit movements of protrusive and retrusive mandibular position. In particular, there are two annular parts (91, 92) inside of which the second end of the first segment (10) is inserted. Said annular parts (91, 92) are adjusted on the first segment (10) by friction so that a first annular part (91) determines the limit of the protrusive position and a second annular part (92) determines the limit of the retrusive mandibular position. To ensure the fit and friction between the first segment (10) and the annular parts (91, 92), these have inner projections (93) of a different material to that of the first segment (10) in order to increase friction.

## Claims

1. A mandibular advancement selector, having:
- A first substantially straight segment, with a first end equipped with attachment means for attaching to a first mouth holder,
- A second segment able to be displaced in parallel with respect to the first segment and having a first end equipped with attachment means for attaching to a second mouth holder, and
- First displacement means for moving the second segment relative to the first segment, **characterised in that** the second segment comprises:
- A substantially straight first base, with a first end and a second end, associated with the first displacement means,
- A curved guide, with an upper end and a lower end, emerging from the first end of the first base from the lower end of the curved guide, and
- A substantially straight second base, with a first end and a second end, joined at its second end to the curved guide by means of second displacement means, so as to be vertically distanced from the first segment, the curvature of the guide being such that, at the position of maximum distance between the second base and the first segment, the second end of the second base is further away from the first segment than the first end of said second base.

2. The selector, according to claim 1, **characterised in that** the second displacement means between the second base and the curved guide, comprise a projection in the second base and consecutive holes in the curved guide to receive said projection, associated with a first digital support at the second end of the second base, and a second digital support at the upper end of the curved guide.

3. The selector, according to claim 1, **characterised in that** the curved guide comprises means for determining the position of the second base with respect to said guide.

4. The selector, according to claim 1, **characterised in that** the first displacement means comprise a sprocket which engages with a rack, the sprocket being associated with a wing nut which controls the rotation of said sprocket in a regulated and controlled manner.

5. The selector, according to claim 4, **characterised in that** the first displacement means comprise a braking/locking mechanism which prevents the rotation of the sprocket and thus the displacement of the second segment with respect to the first segment.

6. The selector, according to claim 5, **characterised in that** the braking/locking mechanism comprises:
- the sprocket associated with the wing nut by means of a pivot of the wing nut inserted in a central housing of the sprocket, thus achieving a coaxial connection between the two,
- a pin, consisting of a first stem which passes through and is associated with the pivot of the wing nut, and which is attached to a second stem, being coaxial with each other and with the sprocket, said pin being movable between two positions on the inside of the wing nut, and
- a friction element located on the second stem,
so that, in one of the two pin positions, the friction element makes contact with an element of the first displacement mechanism, preventing the rotation of the wing nut and therefore the displacement of the second segment with respect to the first segment.

7. The selector, according to claim 4, **characterised in that** the rack is located at the second base of the second segment.

8. The selector according to claim 1, **characterised in that** the attachment means for attaching to a second mouth holder are arranged at the first end of the second base, which is aligned with the first end of the second segment.

9. The selector, according to claim 1, **characterised in that** the first segment comprises a ruler for verifying the offset distance of the second segment with respect to said first segment.

10. The selector according to any one of the preceding claims, **characterised in that** the mouth holders comprise attachment means for attaching to the attachment means for attaching the first and second segments.

11. The selector according to any one of the preceding claims, **characterised in that** the first or second mouth holder is at least one of a tray to receive the jaws or a fork to be placed under the jaws or a support-guide for the incisors or a splint attached to at least one of the two segments.

12. The selector, according to claim 11, **characterised in that** it is a fork or support-guide comprising adaptation means for adapting to the anteroposterior dimensions of the patient's teeth.

13. The selector, according to claim 12, **characterised in that** said adaptation means comprise at least two parallel flanges arranged perpendicularly to a horizontal surface of the fork or support-guide from which the flanges can be separated.

14. The selector, according to claim 11, **characterised in that** it is a tray comprising adaptation means for adapting to the transverse size of the patient's arch.

15. The selector, according to claim 14, **characterised in that** said adaptation means comprise an attachment in the central area of the tray which allows the first and second sides of the tray to be moved closer or further apart from each other.

16. The selector, according to claim 15, **characterised in that** said fastening is a tongue with a lug perpendicular thereto located on one side of said tray which is inserted into at least one of two holes arranged on the other side of said tray.

17. The selector, according to claim 5, **characterised in that** the braking/locking mechanism comprises:
- the sprocket (54) associated with the wing nut (51) by means of a pivot (53) of the wing nut (54) inserted in a central housing of the sprocket (54), thus achieving a coaxial connection between the two,
- a pin, consisting of a first stem (81) which passes through and is associated with the pivot (53) of the wing nut (51) and which is attached to a second stem (84), being coaxial with each other and with the sprocket (54), said pin being movable between two positions on the inside of the wing nut (51), and
- a locking assembly, comprising a ring (82) with teeth therein and a sprocket (85), the toothed ring (82) being located on an element of the first displacement mechanism and the sprocket (85) being arranged on the second stem (84).
